# EUROPÄISCHE PATENTSCHRIFT

(11) **EP 1 001 734 B1**
(45) Veröffentlichungstag und Bekanntmachung des Hinweises auf die Patenterteilung: **22.09.2004**
(21) Anmeldenummer: 98942598.8
(22) Anmeldetag: 22.07.1998
(51) Int. Cl.: A61K 7/13

(54) **FÄRBEMITTEL**
DYES
COLORANTS

(30) Priorität: 31.07.1997 DE 19732975
(43) Veröffentlichungstag der Anmeldung: 24.05.2000
(73) Patentinhaber: Henkel Kommanditgesellschaft auf Aktien, 40589 Düsseldorf (DE)
(72) Erfinder: HÖFFKES, Horst, D-40595 Düsseldorf (DE); SCHRADER, Dieter, D-40589 Düsseldorf (DE); TANAKA, Hiroshi, Saitama Prefecture (JP)
(86) Internationale Anmeldenummer: PCT/EP1998/004604
(87) Internationale Veröffentlichungsnummer: WO 1999/006016

(56) Entgegenhaltungen:
- EP-A- 0 462 857
- EP-A- 0 593 038
- FR-A- 2 636 235
- GB-A- 2 211 517

## Beschreibung

Die Erfindung betrifft Oxidationsfärbemittel zum Färben von Keratinfasern, die spezielle Indolin-Derivate in Kombination mit Kuppler-Komponenten enthalten.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden beispielsweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Amino-pyrazolonderivate sowie 2,4,5,6-Tetraaminopyrimidin und dessen Derivate eingesetzt.

Spezielle Vertreter sind u.a. p-Toluylendiamin, p-Aminophenol, N,N-Bis-(2-hydroxyethyl)-p-phenylendiamin, 2-(2,5-Diaminophenoxy)-ethanol, 1-Phenyl-3-carboxyamido-4-amino-pyrazolon-5 und 4-Amino-3-methylphenol und 2,4,5,6-Tetraaminopyrimidin.

Als Kupplerkomponenten werden in der Regel m-Phenylendiaminderivate, Naphthole, Resorcin und Resorcinderivate, Pyrazolone, m-Aminophenole sowie Pyridin-Derivate verwendet. Als Kupplersubstanzen eignen sich insbesondere 1-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 2,4-Diaminophenoxyethanol, 1,3-Bis-(2,4-diaminophenoxy)-propan, 2-Chlor-resorcin, 2-Chlor-6-methyl-3-aminophenol, 2-Methylresorcin. 2,6-Dihydroxypyridin, 2-Aminomethyl-3-amino-6-methoxy-pyridin und 2,6-Di-aminopyridin.

Bezüglich der in den erfindungsgemäßen Haarfarbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In der Regel gelingt es nicht, mit Hilfe einer einzigen Kuppler/Entwicklerkombination zu natürlichen Farbnuancen zu kommen. In der Praxis ist.daher meist eine Kombination verschiedener Entwicklerkomponenten und Kupplerkomponenten erforderlich. um eine natürlich wirkende Färbung zu erhalten. Gegebenenfalls werden zur Einstellung der Nuance noch zusätzlich sogenannte direktziehende Farbstoffe benötigt.

Es besteht daher ständig Bedarf an neuen, verbesserten Färbemitteln.

In den letzten Jahren wurde bei entsprechenden Untersuchungen die Eignung bestimmter Indolin-Derivate insbesondere für Haarfärbemittel gefunden. So beschreibt die EP-B1-0 530 229 die Verwendung von 5,6-Dihydroxyindolin-Derivaten als Kuppler-Komponente in Oxidationsfärbemitteln. Diese Druckschrift offenbart Färbemittel, die neben den Indolinen übliche Entwickler-Komponenten und/oder direktziehende Farbstoffe enthalten. Mittel, die weiterhin übliche Kuppler-Komponenten enthalten, sind dieser Druckschrift nicht zu entnehmen. Gegenstand der EP-B1-0 613 366 ist die Verwendung der gleichen 5,6-Dihydroxyindolin-Derivate zur Verbesserung von Färbeeigenschaften von Mitteln. die entweder auf Basis von direktziehenden Farbstoffen oder auf Basis von Oxidationsfarbstoffvorprodukten vom Kuppler- und Entwicklertyp formuliert sind. Die Kombination dieser 5,6-Dihydroxyindoline nur mit Oxidationsfarbstoffvorprodukten vom Kuppler-Typ ist der Druckschrift nicht zu entnehmen.

Es wurde nun gefunden, daß mit Oxidationsfärbemitteln, die als Farbstoffvorprodukte diese 5,6-Dihydroxyindolinderivate in Kombination mit üblichen Kuppler-Komponenten enthalten, überraschenderweise exzellente Ausfärbungen mit hervorragenden Echtheitseigenschaften erzielt werden können. Überraschenderweise werden auch eine Vielzahl von Ausfärbungen erzielt, die frei von Rotanteilen sind. Solche, vor allem für dunkles bis schwarzes Haar interessante Ausfärbungen lassen sich mit den bekannten Kuppler-Entwickler-Kombinationen nur unter großen Schwierigkeiten verifizieren. Insbesondere ist es erfindungsgemäß möglich, teilweise bzw. total ergrautes Haar in die ursprüngliche natürliche Nuance so zurückzutönen, daß kein signifikanter Unterschied zu gegebenenfalls noch vorhandenem, natürlich pigmentiertem Haar sichtbar ist. In diesem Zusammenhang sind die vom Fachmann als "matt" bezeichneten Nuancen von besonderer Bedeutung.

Gegenstand der vorliegenden Erfindung sind daher Oxidationsfärbemittel zum Färben von Keratinfasern, insbesondere menschlichen Haaren, enthaltend mindestens eine Kupplerkomponente in einem wäßrigen Träger, die
(a) mindestens ein Derivat des 5,6-Dihydroxyindolins der Formel (I) enthalten, 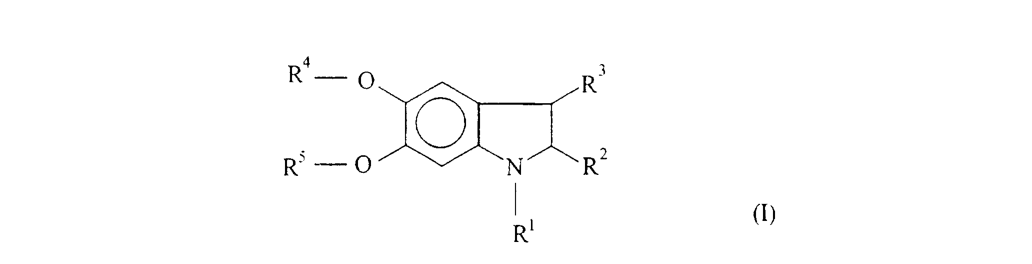 in der unabhängig voneinander
   R¹ steht für Wasserstoff, eine C1-C4-Alkylgruppe oder eine C1-C4-Hydroxyalkylgruppe,
   R² steht für Wasserstoff oder eine -COOH-Gruppe, wobei die -COOH-Gruppe auch als Salz mit einem physiologisch verträglichen Kation vorliegen kann,
   R³ steht für Wasserstoff oder eine C1-C4-Alkylgruppe,
   R⁴ steht für Wasserstoff, eine C1-C4-Alkylgruppe oder eine Gruppe -CO-R⁶, in der
   R⁶ steht für eine C1-C4-Alkylgruppe, und
   R⁵ steht für eine der unter R⁴ genannten Gruppen,
   oder ein physiologisch verträgliches Salz dieser Verbindungen mit einer organischen oder anorganischen Säure, und
(b) frei sind von Oxidationsfarbstoffvorprodukten vom Typ der Entwicklerkomponenten.

Unter Keratinfasern sind dabei Pelze, Wolle, Federn und insbesondere menschliche-Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die erfindungsgemäßen Färbemittel enthalten als erste zwingende Komponente Derivate des 5,6-Dihydroxyindolins gemäß Formel (I). Erfindungsgemäß geeignete Vertreter sind beispielsweise 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin. Bevorzugte Derivate sind das N-Methyl-5,6-dihydroxyindolin sowie insbesondere das 5,6-Dihydroxyindolin.

Die in den erfindungsgemäßen Mitteln enthaltenen Verbindungen der Formel (I) können Sowohl als freie Basen als auch in Form ihrer physiologisch verträglichen Salze mit anorganischen oder organischen Säuren. z.B. der Hydrochloride, der Sulfate und Hydrobromide, eingesetzt werden.

Als zweite zwingende Komponente enthalten die erfindungsgemäßen Färbemittel eine Kuppler-Komponente.

Erfindungsgemäß verwendbare Kuppler-Komponenten sind beispielsweise 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Ami-no-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Bevorzugte Kupplerkomponenten sind 1-Naphthol, Pyrogallol, 1,5-, 2,7- und 1,7-Dihydroxynaphthalin, 5-Amino-2-methylphenol, 5-Amino-2-methyl-4-chlorphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 4-Chlor-resorcin, 2-Chlor-6-methyl-3-aminophenol, 2-Methylresorcin, 5-Methyl-resorcin, 2,6-Dihydroxypyridin, 2,6-Di-aminopyridin, 2,4-Diaminophenoxyethanol, 2-Chlor-6-methyl-3-aminophenol und deren physiologisch verträgliche Salze.

Besonders bevorzugte Kuppler-Komponenten sind 2,4-Diaminophenoxyethanol, Resorcin, 2-Methylresorcin, 2,5-Dimethylresorcin, 4-Chlorresorcin, 5-Amino-2-methylphenol, 5-Amino-2-methyl-4-chlorphenol, m-Aminophenol, 1-Naphthol, 2,7-Dihydroxynaphthalin und deren physiologisch verträgliche Salze.

Selbstverständlich können die erfindungsgemäßen Mittel auch mehr als eine Kuppler-Komponente enthalten. Bevorzugte Kuppler-Kombinationen sind
- 2,4-Diaminophenoxyethanol / 1,3-Bis-(2,4-diaminophenoxy)-propan
- 2,4-Diaminophenoxyethanol / Resorcin
- 2,4-Diaminophenoxyethanol / 2-Methylresorcin
- 2,4-Diaminophenoxyethanol / 5-Methylresorcin
- 2,4-Diaminophenoxyethanol / 4-Chlorresorcin
- 2,4-Diaminophenoxyethanol / Resorcinmonomethylether
- 2,4-Diaminophenoxyethanol / Resorcin / 2-Methylresorcin
- 2,4-Diaminophenoxyethanol / Resorcin / 5-Methylresorcin
- 2,4-Diaminophenoxyethanol / Resorcin / 4-Chlorresorcin
- 2,4-Diaminophenoxyethanol / Resorcinmonomethylether / 4-Chlorresorcin
- 2,4-Diaminophenoxyethanol / Resorcinmonomethylether / 2-Methylresorcin
- 2,4-Diaminophenoxyethanol / Resorcinmonomethylether / 5-Methylresorcin
- 2,4-Diaminophenoxyethanol / 2-Methylamino-3-amino-6-methoxypyridin
- 2,4-Diaminophenoxyethanol / 2,6-Dimethoxy-3,5-diaminopyridin
- 2,4-Diaminophenoxyethanol / 5-Amino-2-methylphenol
- 1,3-Bis-(2,4-diaminophenoxy)-propan / Resorcin
- 1,3-Bis-(2,4-diaminophenoxy)-propan / 2-Methylresorcin
- 1,3-Bis-(2,4-diaminophenoxy)-propan / 5-Methylresorcin
- 1,3-Bis-(2,4-diaminophenoxy)-propan / 4-Chlorresorcin
- 1,3-Bis-(2,4-diaminophenoxy)-propan / Resorcinmonomethylether
- 1,3-Bis-(2,4-diaminophenoxy)-propan / 2-Methylamino-3-amino-6-methoxypyridin
- 1,3-Bis-(2,4-diaminophenoxy)-propan / 2,6-Dimethoxy-3,5-diaminopyridin
- 1,3-Bis-(2,4-diaminophenoxy)-propan / 5-Amino-2-methylphenol
- 2-Methylamino-3-amino-6-methoxypyridin / 2,6-Dimethoxy-3,5-diaminopyridin
- 2-Methylamino-3-amino-6-methoxypyridin / Resorcin
- 2-Methylamino-3-amino-6-methoxypyridin / 2-Methylresorcin
- 2-Methylamino-3-amino-6-methoxypyridin / 5-Methylresorcin
- 2-Methylamino-3-amino-6-methoxypyridin / 4-Chlorresorcin
- 2-Methylamino-3-amino-6-methoxypyridin / Resorcinmonomethylether
- 2-Methylamino-3-amino-6-methoxypyridin/ 5-Amino-2-methylphenol
- 2,6-Dimethoxy-3,5-diaminopyridin / Resorcin
- 2,6-Dimethoxy-3,5-diaminopyridin / 2-Methylresorcin
- 2,6-Dimethoxy-3,5-diaminopyridin / 5-Methylresorcin
- 2,6-Dimethoxy-3,5-diaminopyridin / 4-Chlorresorcin
- 2,6-Dimethoxy-3,5-diaminopyridin / Resorcinmonomethylether
- 2,6-Dimethoxy-3,5-diaminopyridin / 5-Amino-2-methylphenol
- 5-Amino-2-methylphenol / Resorcin
- 5-Amino-2-methylphenol / 2-Methylresorcin
- 5-Amino-2-methylphenol / 5-Methylresorcin
- 5-Amino-2-methylphenol / 4-Chlorresorcin
- 5-Amino-2-methylphenol / Resorcinmonomethylether

Die Kombination Resorcin/2,4-Diaminophenoxyethanol ist eine ganz besonders bevorzugte Kuppler-Kombination.

Die erfindungsgemäßen Oxidationsfärbemittel enthalten die Verbindung der Formel (I) in üblicherweise Mengen von 0,05-10 Gew.-%, vorzugsweise 0.2-5 Gew.-%, und die Kuppler-Komponenten in Mengen von 0,01 bis 20 Gew.-%, vorzugsweise 0.2 bis 5 Gew.-%, jeweils bezogen auf das gesamte Oxidationsfärbemittel.

Es ist erfindungsgemäß bevorzugt, daß die Färbemittel neben den 5,6-Dihydroxyindolin-Derivaten gemäß Formel (I) und den Kupplerkomponenten keine weiteren Farbstoffe bzw. Farbstoff-Vorprodukte enthalten. Es soll jedoch nicht ausgeschlossen werden, daß die erfindungsgemäßen Färbemittel, insbesondere zur leichten Nuancierung und in untergeordneten Mengen, zusätzlich direktziehende Farbstoffe enthalten können. Direktziehende Farbstoffe sind üblicherweise Nitrophenylendiamine, Nitroaminophenole. Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN. Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitrotoluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Unter diesen genannten direktziehenden Farbstoffen sind Verbindungen, die blaue Farbtöne ergeben, bevorzugt. Mittel gemäß dieser Ausführungsform enthalten die direktziehenden Farbstoffe bevorzugt in einer Menge von mehr als 0,01 Gew.-%, bezogen auf das gesamte Färbemittel.

Weiterhin können die erfindungsgemäßen Färbemittel auch in der Natur vorkommende-Farbstoffe wie beispielsweise Henna rot, Henna neutral. Henna schwarz. Kamillenblüte, Sandelholz, schwarzer Tee, grüner Tee, Faulbaumrinde, Salbei, Blauholz, Krappwurzel, Catechu, Sedre und Alkannawurzel enthalten. Gerbstoffe enthaltende Produkte wie Henna neutral und grüner Tee sind bevorzugt.

Es ist nicht erforderlich, daß die 5,6-Dihydroxyindolin-Derivate, die Kupplerkomponenten sowie die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel werden bevorzugt auf einen pH-Wert von 6,5 bis 11.5, insbesondere von 9 bis 10, eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Diund Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbin-dungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine
- COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethylammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden. die außer einer C8-C18-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyl-iminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methyl-hydroxyalkyl-dialkoyloxyalkyl-ammonium-methosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/ Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- Alkalisierungsmittel wie beispielsweise Ammoniak, Monoethanolamin, 2-Amino-2-methylpropanol, 2-Amino-2-methyl-propandiol-1,3, Triethanolamin, Alkali- und Erdalkalimetallhydroxide,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft,
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0.1 bis 25 Gew.-% des gesamten Färbemittels eingesetzt.

Gemäß einer ersten, bevorzugten Ausführungsform erfolgt die oxidative Entwicklung der Färbung allein mit Luftsauerstoff. Dazu wird das erfindungsgemäße Mittel auf das Haar aufgetragen, dort bevorzugt 5 bis 30 Minuten belassen und anschließend ausgespült. Gewünschtenfalls kann das Haar dann noch shampooniert werden.

Gemäß einer zweiten Ausführungsform kann zusätzlich noch ein chemisches Oxidationsmittel eingesetzt werden. Dies ist besonders dann vorteilhaft, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat in Frage. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Mengen vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken. Zweckmäßigerweise wird die Zubereitung des Oxidationsmittels unmittelbar vor dem Färben der Haare mit der Zubereitung mit den Oxidationsfarbstoffvorprodukten vermischt. Das dabei entstehende gebrauchsfertige Haarfärbepräparat sollte bevorzugt einen pH-Wert im Bereich von 5 bis 11, insbesondere 6 bis 10, aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 5 bis 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Oxidationsfarbstoffvorprodukten ohne vorherige Vermischung mit der Oxidationskomponente auf das Haar aufgebracht werden. Nach einer Einwirkdauer von 5 bis 30, insbesondere 20 bis 30. Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert.

Unabhängig davon, welches der oben genannten Verfahren zur Anwendung des erfindungsgemäßen Mittels gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Zinksulfat ist ein besonders bevorzugtes Metallsalz. Durch Verwendung dieser Metallsalze kann sowohl die Ausbildung der Färbung beschleunigt als auch die Farbnuance gezielt beeinflußt werden.

Überraschenderweise hat sich gezeigt, daß abhängig von der Wahl des Oxidationsverfahrens unterschiedliche Färbeergebnisse mit dem gleichen Färbemittel erhalten werden können.

Gegenstand der Anmeldung ist daher auch ein Verfahren zum Färben menschlicher Haare, bei dem eines der oben genannten erfindungsgemäßen Mittel auf das Haar aufgebracht wird und anschließend die Ausfärbung erfolgt. Dabei kann die Ausbildung der Farbe mittels Luftsauerstoff bevorzugt sein.

Gemäß einer besonderen Ausführungsform dieses Verfahrens erfolgt die Ausbildung der endgültigen Färbung durch mehrmaliges Aufbringen des Mittels und jeweils anschließender Luftoxidation. Das jeweilige Aufbringen des Mittels erfolgt dabei bevorzugt im Abstand von jeweils etwa einem Tag. Dadurch können sehr gezielt spezielle Nuancen erhalten werden.

Die nachfolgenden Beispiele sollen den Erfindungsgegenstand näher erläutern.

### Beispiele

### 1. Ausfärbungen

Es wurden zunächst Färbemittel mit den in Tabelle 1 aufgeführten Zusammensetzungen hergestellt [alle Angaben sind, soweit nicht anders vermerkt, in g].

Bei der Ausfärbung mit H₂O₂-Oxidation wurde das Färbemittel unmittelbar vor der Anwendung mit einer 3%igen Wasserstoffperoxid- Zubereitung im Massen-Verhältnis 1:1 vermischt. Die erhaltene Anwendungsmischung wurde 30 Minuten bei Raumtemperatur auf dem Haar belassen. Anschließend wurde das Haar gespült und getrocknet.

Bei der Luftoxidation wurde das Färbemittel 30 Minuten bei Raumtemperatur auf dem Haar belassen. Anschließend wurde das Haar gespült und getrocknet.

Die Ausfärbung erfolgte jeweils auf naturweißen Haarsträhnen (Fa. Kerling).

**Tabelle 1:**

| Rezepturen | | |
|---|---|---|
| Komponente | Rezeptur B 1 | Rezeptur V 1 |
| • Texapon® N28¹ | 20,0 | 20,0 |
| • Dehyton®K² | 12,5 | 12,5 |
| • Lorol®techn³ | 2,0 | 2,0 |
| • Hydrenol®D⁴ | 8,5 | 8,5 |
| • Eumulgin®B 2⁵ | 1,5 | 1,5 |
| • 5,6-Dihydroxyindolinhydrobromid | 1,0 | 1,0 |
| • 2,4-Diaminophenoxyethanol | 0,72 | - |
| • Ammoniak | <-----ad pH 9,5 -----> | |
| • Wasser | <-----ad 100 -----> | |

| | | |
|---|---|---|
| ¹ Natriumlaurylethersulfat (ca. 28 % Aktivsubstanz; CTFA-Bezeichnung: Sodium Laureth Sulfate) (HENKEL) | | |
| ² Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (HENKEL) | | |
| ³ C₁₂₋₁₈-Fettalkohol (HENKEL) | | |
| ⁴ C₁₆₋₁₈-Fettalkohol (HENKEL) | | |
| ⁵ Cetylstearylalkohol mit ca. 20 Mol EO (CTFA-Bezeichnung: Ceteareth-20) (HENKEL) | | |

**Tabelle 2:**

| Ausfärbungen | | |
|---|---|---|
| Rezeptur | Ausfärbungsverfahren | Ergebnis |
| B1 | Luftoxidation | mattes mittelblond (ohne Rotstich) |
| V1 | Luftoxidation | rötliches hellblond |
| B 1 | H₂O₂-Oxidation | rötliches dunkelblond |
| V1 | H₂O₂-Oxidation | rötliches mittelblond |

Die Ergebnisse weiterer Ausfärbungen mit Mitteln entsprechend denen gemäß Tabelle 1, die jeweils 2 g 5,6-Dihydroxyindolinhydrobromid und eine äquimolare Menge der angegebenen Kuppler-Komponente enthielten, sind in Tabelle 3 angegeben.

**Tabelle 3:**

| Ausfärbungen | | |
|---|---|---|
| Kuppler-Komponente | Ausfärbungsverfahren | Ergebnis |
| 2,4-Diaminophenoxyethanol | Luftoxidation | mattes dunkelblond |
| 2,4-Diaminophenoxyethanol | H₂O₂-Oxidation | kastanienfarbiges hellbraun |
| 1,3-Bis-(2,4-diaminophenoxy)-propan | Luftoxidation | kupferfarbiges mittelblond |
| 1,3-Bis-(2,4-diaminophenoxy)- | H₂O₂-Oxidation | kupferfarbiges dunkelblond |
| propan | | |
| 2-Methylamino-3-amino-6- | Luftoxidation | kastanienfarbiges mittelbraun |
| methoxypyridin | | |
| 2-Methylamino-3-amino-6- | H₂O₂-Oxidation | matt-kastanienfarbiges |
| methoxypyridin | | dunkelblond |
| 2,6-Dimethoxy-3,5-diamino- | Luftoxidation | kastanienfarbiges hellbraun |
| pyridin | | |
| 2,6-Dimethoxy-3,5-diamino | H₂O₂-Oxidation | kastanienfarbigesmittelbraun |
| pyridin | | |
| Resorcin | Luftoxidation | matt-kastanienfarbiges mittelbraun |
| Resorcin | H₂O₂-Oxidation | kastanienfarbiges mittelbraun |
| 5-Amino-2-methylphenol | Luftoxidation | mattes dunkelblond |
| 5-Amino-2-methyl-phenol | H₂O₂-Oxidation | kastanienfarbiges hellbraun |

Die Ergebnisse weiterer Ausfärbungen, wobei Mittel entsprechend denen gemäß Tabelle 1 zum Einsatz kamen, die jeweils 2 g 5,6-Dihydroxyindolinhydrobromid und eine äquimolare Menge der angegebenen Kuppler-Komponente enthielten, sind in Tabelle 4 angegeben. "1fache" Luftoxidation bedeutet hier die einmalige Anwendung eines Mittels wie oben beschrieben. "3fache" Luftoxidation bedeutetet die dreimalige Anwendung des Mittels wie oben beschrieben an drei aufeinanderfolgenden Tagen.

**Tabelle 4:**

| sukzessive Ausfärbungen mit Luftoxidation | | |
|---|---|---|
| Kuppler-Komponente | Ausfärbungsverfahren | Ergebnis |
| 2,4-Diaminophenoxyethanol | 1fache Luftoxidation | mattes dunkelblond |
| 2,4-Diaminophenoxyethanol | 3fache Luftoxidation | sehr mattes mittelbraun |
| 1,3-Bis-(2,4-diaminophenoxy)- | 1fache Luftoxidation | kupferfarbiges mittelblond |
| propan | | |
| 1,3-Bis-(2,4-diaminophenoxy)- | 3fache Luftoxidation | leicht rötliches hellbraun |
| propan | | |
| 2-Methylamino-3-amino-6- | 1fache Luftoxidation | kastanienfarbiges mittelbraun |
| methoxypyridin | | |
| 2-Methylamino-3-amino-6- | 3fache Luftoxidation | leicht rötliches |
| methoxypyridin | | mittel-dunkelbraun |
| Resorcin | 1fache Luftoxidation | matt-kastanienfarbiges mittelbraun |
| Resorcin | 3fache Luftoxidation | sehr mattes mittelbraun |
| 5-Amino-2-methylphenol | 1fache Luftoxidation | mattes dunkelblond |
| 5-Amino-2-methyl-phenol | 3fache Luftoxidation | sehr mattes, leicht grünstichiges dunkelblond |

## Patentansprüche

1. Oxidationsfärbemittel zum Färben von Keratinfasern, insbesondere menschlichen Haaren, enthaltend mindestens eine Kupplerkomponente in einem wäßrigen Träger, **dadurch gekennzeichnet, daß** es
(a) mindestens ein Derivat des 5,6-Dihydroxyindolins der Formel (I) enthält, 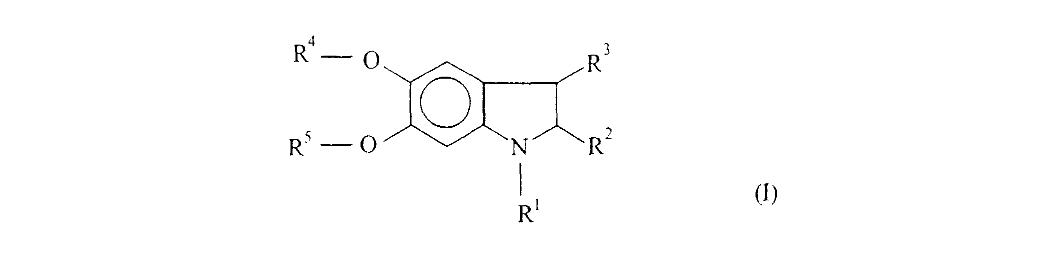 in der unabhängig voneinander
R¹ steht für Wasserstoff, eine C1-C4-Alkylgruppe oder eine C1-C4-Hydroxyalkylgruppe,
R² steht für Wasserstoff oder eine -COOH-Gruppe, wobei die -COOH-Gruppe
auch als Salz mit einem physiologisch verträglichen Kation vorliegen kann,
R³ steht für Wasserstoff oder eine C1-C4-Alkylgruppe,
R⁴ steht für Wasserstoff, eine C1-C4-Alkylgruppe oder eine Gruppe -CO-R⁶, in der
R⁶ steht für eine C1-C4-Alkylgruppe, und
R⁵ steht für eine der unter R⁴ genannten Gruppen,
oder ein physiologisch verträgliches Salz dieser Verbindungen mit einer organischen order anorganischen Säure, und
(b) frei ist von Oxidationsfarbstoffvorprodukten vom Typ der Entwicklerkomponenten.

2. Oxidationsfärbemittel nach Anspruch 1, **dadurch gekennzeichnet, daß** die Verbindung gemäß Formel (I) ausgewählt ist aus 5,6-Dihydroxyindolin, N-Methyl-5,6-dihydroxyindolin sowie deren physiologisch verträglichen Salzen.

3. Oxidationsfärbemittel nach Anspruch 1 oder 2, **dadurch gekennzeichnet, daß** die Kuppler-Komponente ausgewählt ist aus 1-Naphthol, Pyrogallol, 1,5-, 2,7- und 1,7-Dihydroxynaphthalin, 5-Amino-2-methylphenol, 5-Amino-2-methyl-4-chlorphenol, m-Aminophenol, Resorcin. Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlor-resorcin, 2-Chlor-6-methyl-3-aminophenol, 2-Methylresorcin, 5-Methyl-resorcin, 2,6-Dihydroxypyridin, 2,6-Diaminopyridin, 2,4-Diaminophenoxyethanol und deren physiologisch verträglichen Salzen.

4. Oxidationsfärbemittel nach Anspruch 3, **dadurch gekennzeichnet, daß** die Kupplerkomponente ausgewählt ist aus 2,4-Diaminophenoxyethanol, Resorcin, 2-Methylresorcin, 2,5-Dimethylresorcin, 4-Chlorresorcin, 5-Amino-2-methylphenol, 5-Amino-2-methyl-4-chlorphenol, 2-Chlor-6-methyl-3-aminophenol, m-Aminophenol, 1-Naphthol, 2,7-Dihydroxynaphthalin und deren physiologisch verträglichen Salzen.

5. Oxidationsfärbemittel nach einem der Ansprüche 1 bis 5, **dadurch gekennzeichnet, daß** die Verbindung der Formel (I) in Mengen von 0,05-10 Gew.-%. vorzugsweise 0.2-5 Gew.-%, und die Kuppler-Komponenten in einer Menge von 0,01 bis 20 Gew.-%, vorzugsweise 0,2 bis 5 Gew.-%, jeweils bezogen auf das gesamte Oxidationsfärbemittel, enthalten sind.

6. Verwendung eines Mittels nach einem der Ansprüche 1 bis 5 zum Färben von menschlichen Haaren.

7. Verfahren zum Färben menschlicher Haare mit einem Mittel nach einem der Ansprüche 1 bis 5, **dadurch gekennzeichnet, daß** die Ausbildung der Farbe mit Luftsauerstoff erfolgt.

8. Verfahren zum Färben menschlicher Haare mit einem Mittel nach einem der Ansprüche 1 bis 5, **dadurch gekennzeichnet, daß** die Ausbildung der endgültigen Färbung durch mehrmaliges Aufbringen des Mittels und jeweils anschließender Luftoxidation erfolgt.

## Claims

1. An oxidation colorant for coloring keratin fibers, more particularly human hair, containing at least one secondary intermediate in an aqueous carrier, **characterized in that** it contains
(a) at least one derivative of 5,6-dihydroxyindoline corresponding to formula (I): 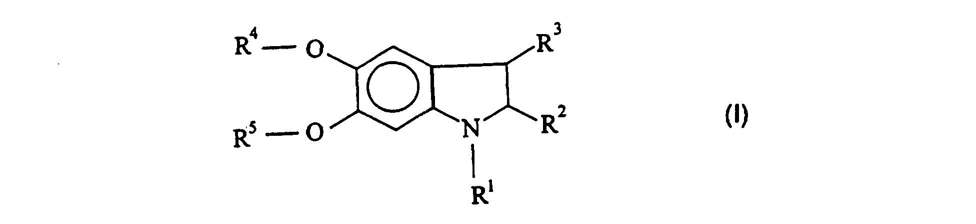 in which - independently of one another -
R¹ is hydrogen, a C₁₋₄ alkyl group or a C₁₋₄ hydroxyalkyl group,
R² is hydrogen or a -COOH group which may even be present as a salt with a physiologically compatible cation,
R³ is hydrogen or a C₁₋₄ alkyl group,
R⁴ is hydrogen, a C₁₋₄ alkyl group or a group -CO-R⁶ where R⁶ is a C₁₋₄ alkyl group and
R⁵ is one of the groups mentioned for R⁴,
or a physiologically compatible salt of these compounds with an organic or inorganic acid, and
(b) is free from oxidation dye precursors of the primary intermediate type.

2. An oxidation colorant as claimed in claim 1, **characterized in that** the compound corresponding to formula (I) is selected from 5,6-dihydroxyindoline, N-methyl-5,6-dihydroxyindoline and physiologically compatible salts thereof.

3. An oxidation colorant as claimed in claim 1 or 2, **characterized in that** the secondary intermediate is selected from 1-naphthol, pyrogallol, 1,5-, 2,7-and 1,7-dihydroxynaphthalene, 5-amino-2-methylphenol, 5-amino-2-methyl-4-chlorophenol, m-aminophenol, resorcinol, resorcinol monomethyl ether, m-phenylenediamine, 1-phenyl-3-methyl-5-pyrazolone, 2,4-dichloro-3-aminophenol, 1,3-bis-(2,4-diaminophenoxy)-propane, 4-chlororesorcinol, 2-chloro-6-methyl-3-aminophenol, 2-methyl resorcinol, 5-methyl resorcinol, 2,6-dihydroxypyridine, 2,6-diaminopyridine, 2,4-diaminophenoxyethanol and physiologically compatible salts thereof.

4. An oxidation colorant as claimed in claim 3, **characterized in that** the secondary intermediate is selected from 2,4-diaminophenoxyethanol, resorcinol, 2-methyl resorcinol, 2,5-dimethyl resorcinol, 4-chlororesorcinol, 5-amino-2-methylphenol, 5-amino-2-methyl-4-chlorophenol, m-aminophenol, 1-naphthol, 2,7-dihydroxynaphthalene and physiologically compatible salts thereof.

5. An oxidation colorant as claimed in any of claims 1 to 5, **characterized in that** the compound corresponding to formula (I) is present in quantities of 0.05 to 10% by weight and preferably 0.2 to 5% by weight while the secondary intermediates are present in a quantity of 0.01 to 20% by weight and preferably 0.2 to 5% by weight, based on the oxidation colorant as a whole.

6. The use of the colorant claimed in any of claims 1 to 5 for coloring human hair.

7. A process for coloring human hair with the colorant claimed in any of claims 1 to 5, **characterized in that** the color is developed with atmospheric oxygen.

8. A process for coloring human hair with the colorant claimed in any of claims 1 to 5, **characterized in that** the final color is developed by repeated application of the colorant and subsequent oxidation with air.

## Revendications

1. Colorant par oxydation pour colorer les fibres de kératine, en particulier les cheveux humains, contenant au moins un composant de couplage dans un support aqueux, **caractérisé en ce qu'**il contient
(a) au moins un dérivé de 5,6-dihydroxyindoline de formule (I) 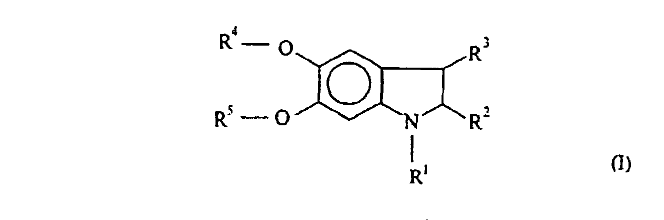 dans laquelle, indépendamment l'un de l'autre
R¹ représente un hydrogène, un groupe alkyle en C₁ à C₄ ou un groupe hydroxyalkyle en C₁ à C₄,
R² représente un hydrogène ou un groupe -COOH, où le groupe -COOH peut également se présenter sous forme de sel avec un cation physiologiquement acceptable,
R³ représente un hydrogène ou un groupe alkyle en C₁ à C₄,
R⁴ représente un hydrogène, un groupe alkyle en C₁ à C₄ ou un groupe -CO-
R⁶, où R⁶ représente un groupe alkyle en C₁ à C₄, et
R⁵ représente l'un des groupes mentionnés sous R⁴,
ou un sel physiologiquement acceptable de ces composés avec un acide organique ou inorganique, et
(b) **en ce qu'**il est dépourvu de précurseurs de colorants par oxydation du type des composants révélateurs.

2. Colorant par oxydation selon la revendication 1, **caractérisé en ce que** le composé selon la formule (I) est choisi parmi la 5,6-dihydroxyindoline, la N-méthyl-5,6-dihydroxyindoline ainsi que leurs sels physiologiquement acceptables.

3. Colorant par oxydation selon la revendication 1 ou 2, **caractérisé en ce que** le composant de couplage est choisi parmi le 1-naphtol, le pyrogallol, le 1,5-, le 2,7- et le 1,7-dihydroxynaphtalène, le 5-amino-2-méthylphénol, le 5-amino-2-méthyl-4-chlorophénol, le m-aminophénol, la résorcine, l'éther monométhylique de résorcine, la m-phénylènediamine, la 1-phényl-3-méthyl-pyrazolone-5, le 2,4-dichloro-3-aminophénol, le 1,3-bis-(2,4-diaminophénoxy)-propane, la 4-chloro-résorcine, le 2-chloro-6-méthyl-3-aminophénol, la 2-méthylrésorcine, la 5-méthyl-résorcine, la 2,6-dihydroxypyridine, la 2,6-diaminopyridine, le 2,4-diaminophénoxyéthanol et leurs sels physiologiquement acceptables.

4. Colorant par oxydation selon la revendication 3, **caractérisé en ce que** le composant de couplage est choisi parmi le 2,4-diaminophénoxyéthanol, la résorcine, la 2-méthyl-résorcine, la 2,5-diméthylrésorcine, la 4-chlororésorcine, le 5-amino-2-méthylphénol, le 5-amino-2-méthyl-4-chlorophénol, le 2-chloro-6-méthyl-3-aminophénol, le m-aminophénol, le 1-naphtol, le 2,7-dihydroxynaphtalène et leurs sels physiologiquement acceptables.

5. Colorant par oxydation selon l'une des revendications 1 à 5, **caractérisé en ce que** le composé de formule (I) est contenu à des quantités de 0,05 à 10 % en poids, de préférence de 0,2 à 5 % en poids, et **en ce que** le composant de couplage est contenu en une quantité de 0,01 à 20 % en poids, de préférence de 0,2 à 5 % en poids, toujours par rapport à l'ensemble du colorant par oxydation.

6. Utilisation d'un agent selon l'une des revendications 1 à 5 pour la coloration des cheveux humains.

7. Procédé de coloration des cheveux humains avec un agent selon l'une des revendications 1 à 5, **caractérisé en ce que** la formation de la couleur s'effectue avec l'oxygène de l'air.

8. Procédé de coloration des cheveux humains avec un agent selon l'une des revendications 1 à 5, **caractérisé en ce que** la formation de la coloration finale s'effectue par application de l'agent en plusieurs fois, puis à chaque fois oxydation par l'air.
